# EUROPEAN PATENT SPECIFICATION

(11) **EP 4 436 652 B1**
(45) Date of publication and mention of the grant of the patent: **12.08.2026**
(21) Application number: 22821395.5
(22) Date of filing: 21.11.2022
(51) Int. Cl.: A61N 1/36

(54) **STIMULATION THERAPY WITH REDUCED ENERGY**
STIMULATIONSTHERAPIE MIT REDUZIERTER ENERGIE
THÉRAPIE DE STIMULATION À ÉNERGIE RÉDUITE

(30) Priority: 23.11.2021 US 202163282221 P; 07.12.2021 EP 21212759
(43) Date of publication of application: 02.10.2024
(73) Proprietor: BIOTRONIK SE & Co. KG, 12359 Berlin (DE)
(72) Inventor: BARU, Marcelo, Tualatin, Oregon 97062 (US); KIBLER, Andrew B., Lake Oswego, Oregon 97035 (US)
(74) Representative: Biotronik Corporate Services SE
(86) International application number: PCT/EP2022/082607
(87) International publication number: WO 2023/094315

(56) References cited:
- US-A1- 2006 173 493
- US-A1- 2018 272 124
- US-A1- 2019 329 025
- US-A1- 2019 329 041
- US-B2- 7 801 615
- US-B2- 9 937 344

## Description

The invention is directed to a system device for stimulation therapy, in particular for neurostimulation.

Neurostimulation devices are used to deliver electrical stimulation therapy to a patient's body to various tissue sites to treat a variety of symptoms or conditions such as chronic pain, Parkinson's disease, epilepsy, urinary or fecal incontinence, sexual dysfunction, obesity or gastroparesis. Such devices usually deliver electrical stimulation therapy via one or more leads that comprise electrodes located proximate to target locations associated with the brain, the spinal cord, pelvic nerves, peripheral nerves, or the gastrointestinal tract of the patient. Hence, electrical simulation may be used in different therapeutic applications, such as deep brain stimulation (DBS), spinal cord stimulation (SCS), pelvic stimulation, gastric stimulation, or peripheral nerve stimulation (PNS).

Spinal cord stimulation (SCS), as a means of pain relief for patients suffering from neuropathic pain, has traditionally been thought of as requiring paresthesia sensations to overlap a patient's region of pain in order to provide relief. Recent research has shown that an alternate paresthesia-free mechanism of pain relief is available through higher frequency (compared to traditional tens of Hz) stimulation which is effective in patients without requiring intra-operative electrode mapped selection.

In the last few years, therapies have demonstrated efficacy of a paresthesia-free method of pain relief whereby the patient does not experience sensations associated with electrical stimulation and the stimulation electrodes selected may not map directly to a dermatomal alignment with the patient's region of pain. High-frequency SCS therapy utilizes stimulation frequencies between 1.5 kHz and 100 kHz, for example 10 kHz, to achieve a neuromodulatory effect without recruiting the dorsal column fibers associated with paresthesia. Research indicates that this therapy modality reduces the wind-up hypersensitivity of dorsal horn interneurons responsible for relaying a painful sensation from the peripheral to the central nervous system. Pain relief associated with this stimulation may require several hours to a day to take effect.

The mechanism of action of this mode of therapy is still under debate; however; the prevailing theory is as follows. High-frequency SCS stimulation has little influence on the dorsal column axons which facilitate paresthesia therapy, instead directly inducing slight potentiation changes on lamina I neurons in the dorsal horn of the spinal cord. The potentiation changes trigger a cascade of intracellular signalling responses which induce a direct inhibition of sensitization and suppression of activity of neuropathic pain relay neurons in the dorsal horn.

This paresthesia-free SCS approach is similar in frequencies to high-frequency transcutaneous spinal electroanalgesia (TSE) which has been available for decades. Whether the underlying mechanisms and site of pain relief action are the same between high-frequency TSE and high-frequency SCS remains to be determined. Further, in document US Patent 10,870,000 an SCS approach is disclosed which is able to achieve efficient pain relief with stimulation frequencies below or equal to 1.5 kHz. The known SCS device utilizes rectangular stimulation pulses which tend to influence the largest diameter fibers which are associated with paresthesia. However, larger diameter nerve fibres have lower stimulation thresholds than smaller ones. This implies that in a span of low stimulation amplitude to high, the former will be stimulated first when located at the same distance from a stimulation site or, in other words, the recruitment order is weighted toward activation of large diameter fibres. This presents a challenge to increasing the charge injected to recruit deeper and/or smaller diameter nerve fibers that might further increase the efficacy of a sub-perception therapy.

Patent application US 2019/329041 A1 describes a medical device which is configured to deliver a series of electrical stimulation pulses including opposing polarity pulses. The medical device delivers a charge balancing pulse by modifying every nth pulse of the electrical stimulation pulses to reduce a net charge delivered over the series of electrical stimulation pulses.

US 9,937,344 B2 relates to systems, devices, and techniques for delivering electrical stimulation therapy to a patient. In one example, the disclosure relates to a method including delivering a series of pulses with alternating pulse polarities to a gastrointestinal tract of a patient. The series of pulses includes at least a first pulse of a first polarity, a second pulse of a second polarity, and a third pulse of the first polarity, where the first, second and third pulses are delivered in direct succession and in that order.

US 2019/329025 A1 describes medical device systems, methods, and algorithms are disclosed for providing complex stimulation waveforms. The waveforms may selectively modulate or activate specific neural targets or selected ratios of specific neural targets. Some of the waveforms include pre-pulse phases defined by parameters, the value of which changes during the pre-pulse phase.

US 7,801,615 B2 discloses implantable pulse generators and spinal cord stimulation (SCS) systems. The implantable pulse generator comprises telemetry circuitry configured for wirelessly receiving programming signals from an external programmer, and a plurality of bi-directional current sources configured for being respectively coupled to a plurality of electrodes. The implantable pulse generator further comprises control circuitry configured for directing the bi-directional current sources to output current pulses of a selected amplitude and polarity to the electrodes in accordance with the programming signals.

US 2006/0173493 A1 describes a method, system, and an apparatus for providing a multi-phasic stimulation signal for an implantable device. An electrical pulse with a first characteristic that includes a first pulse width, a first pulse amplitude, a first pulse polarity, or a first pulse shape, is applied during a first time period to a portion of a vagus nerve using an implantable device.

Accordingly, there is a desire to provide a system and/or a device and method that deliver a sub-perception neurostimulation therapy arresting the propagation of action potentials of large-diameter fibers to recruit deeper and/or smaller diameter nerve fibers that might increase the therapy efficacy.

The above object is solved by a system for neurostimulation of a patient's body with the features of claim 1. The invention is defined in the appended claims. Aspects, embodiments and examples disclosed herein which do not fall within the scope of the appended claims do not form part of the invention, and are merely provided for illustrative purposes.

In the following the invention is described as a system, but it could also be viewed or considered as a device. If the invention is considered as a device, in the following the term 'system' needs to be replaced by the term 'device' and the term 'device' needs to be replaced by the term 'pulse generator'.

In particular, the above object is solved by a system for neurostimulation of a patient's body, wherein the system comprises a plurality of Z electrodes and a device, wherein for the number of the plurality of electrodes Z ≥ 3 applies (or wherein the number the plurality of electrodes Z is equal to or higher than 3), wherein the device is configured to deliver via each electrode of a group of N electrodes (N ≤ Z and if Z = 3 then N = Z) a set of pulses including one therapeutic electric pulse having an amplitude I1, I2, ... IN and a number of (N-1) charge balancing electric pulses wherein the charge balancing electric pulses each have a polarity being opposite a polarity of the therapeutic electric pulse, and wherein each therapeutic electric pulse and each charge balancing electric pulse has a rampart or non-rectangular shape.

This shape may have a leading edge, a trailing edge and a plateau between the leading edge and the trailing edge, wherein at least one section of the leading edge and/or the trailing edge forms a linear curve or an exponential curve, and wherein the plateau has a changing or constant amplitude.

The above neurostimulation system comprises a plurality of Z electrodes, wherein all or a (sub-)group of N (i.e. N is less than or equal to Z) electrodes of these electrodes is used for delivery of a therapeutic electric pulse. Z is equal to or larger than 3 and if Z = 3 then N = Z. The neurostimulation therapy is delivered in cycles, wherein each cycle has several electrical phases in which one electrode provides a therapeutic electric pulse and the (N-1) other electrodes provide charge balancing electric pulses. At the end of each cycle, an additional passive balance phase may be incorporated to allow each electrode to return to a baseline low level of charge before the stimulation cycle repeats. The plurality of electrodes may be located at a distal portion of at least one percutaneous lead implantable in vicinity of a target for stimulation within the patient's body. Alternatively, the lead may be a paddle lead (surgical lead) or any other type of lead for neurostimulation. The device is or may comprise a pulse generator which may be implantable. The pulse generator comprises a connection module for electrically connecting the at least one lead to its electrical circuitry. The pulse generator comprises the electrical circuitry, for example comprising an application specific integrated circuit (ASIC), for providing stimulation output and a power supply such as a battery, wherein the electrical circuitry, the power supply and the at least one lead with the electrodes are electrically interconnected with each other. The pulse generator may comprise a processor and/or a memory for storing data and/or a communication module for communication with an external computer. The processor, memory and/or communication module may be electrically interconnected with the electrical circuitry, the power supply and the at least one lead. All elements of the pulse generator are contained within a hermetically sealed housing.

The group of N electrodes of the plurality of electrodes can also be or be called a subset of electrodes of the plurality of Z electrodes.

The one electrode delivering the therapeutic electric pulse of stimulation in one phase of the cycle, whether it is anodic or cathodic in nature, is delivering a higher primary current than the return current (balancing pulses) delivered by the greater number (N-1) of other individual electrodes participating in the stimulation. This higher primary current will induce a bias in the medium-distance field of stimulation, driving cellular response to be primarily hyperpolarizing or primarily depolarizing in nature. In the above approach a similar bias is accomplished but distributed among the other (N-1) electrodes which are participating in the stimulation waveform cycle. A passive balance between all participating electrodes, preferably before each stimulation cycle repeats, prevents voltage runaway in the effective series capacitances of the stimulation paths. In one embodiment the charge balancing electric pulses for charge balancing in one phase have in sum the same amount of charge as the therapeutic electric pulse of the same phase.

In one embodiment, the plateau has the changing amplitude, and the changing amplitude comprises at least two periodic oscillations between a first amplitude value and a second amplitude value, the absolute values of the first and the second amplitude values are greater than zero and the first amplitude value and the second amplitude value have the same sign.

In one embodiment, a current of the therapeutic electric pulse is equal to a sum of currents of the charge balancing electric pulses, which are preferably provided in the same phase of the cycle as the therapeutic electric pulse.

In one embodiment, each of the N electrodes undergoes a recurring pattern of a therapeutic electric pulse (Primary Phase) with a current amplitude I and a series of (N-1) charge balancing electrical pulses (Secondary Phases), which pass an inverted current amplitude I of the therapeutic electric pulse, in one embodiment distributed with equal weight (I/(N-1)). The therapeutic electric pulse and the charge balancing electric pulses are separated by one inter-pulse interval. Further, each therapeutic electric pulse may be timely aligned with one charge balancing electric pulse of the other (N-1) electrodes such that in the system only one therapeutic electric pulse occurs at a time. The charge balancing electrodes serve as return electrodes. After every electrode cyclically provided one therapeutic electric pulse the cycle starts again with the first one of the N electrodes.

According to the invention, each therapeutic electric pulse and each charge balancing electric pulse may have a rampart or non-rectangular shape with a leading edge, a trailing edge and a plateau between the leading edge and the trailing edge, wherein at least a section of the leading edge and/or the trailing edge forms a linear curve or an exponential curve, wherein the plateau has a changing or constant amplitude. The changing amplitude may be a periodically changing amplitude. This means that each electrode provides the electric pulses (i.e. the therapeutic electric pulses and the charge balancing electric pulses) having the above-defined rampart or non-rectangular shape which is a shape that is different from a spike shape (e.g. a capacitive discharge in tissue or switched-capacitor stimulator) because its plateau extends over a pre-defined plateau time period. At least one of the leading and the trailing edge of each therapeutic electric pulse and each charge balancing electric pulse does not have any fast edge shape(e.g. more than a few µs) but increases or decreases linearly or exponentially, respectively, at least over a section of the edge. In one embodiment, the linear or exponential course extends over the full length of the leading edge and/or of the trailing edge.

In one embodiment, the rampart or non-rectangular shape of the charge balancing electric pulse comprises
a) the leading edge having (either) a fast (rising) edge shape (e.g. a few µs settling time), an exponential shape or a linear shape, and
b) the trailing edge having (either) a linear shape (linear slope) or a section running exponentially (exponential shape).

In one embodiment, each therapeutic electric pulse and each charge balancing electric pulse has the rampart shape, wherein the rampart shape of the charge balancing electric pulse comprises
a) the leading edge having a rectangular shape, an exponential shape or a linear shape, and
b) the trailing edge having a linear shape or a section running exponentially.

The plateau of the pulse is located between the end of the leading edge and the beginning of the trailing edge of each electric pulse and has an amplitude which is significantly different from zero over the pre-defined plateau time period. The amplitude may be a constant value (i.e. different from zero) during the whole plateau time period or may change between the leading edge and the trailing edge, for example periodically, thereby forming a periodic function. In the latter example and in another example/embodiment above, the changing amplitude may comprise at least two oscillations (i.e. two fundamental periods) between a first amplitude value and a second amplitude value, wherein the absolute values of the first and the second amplitude values are greater than zero and the first amplitude value and the second amplitude value have the same sign, i.e. both amplitude values are either positive or negative. In one embodiment one section of the fundamental period of the periodic function is an exponential decay. Such form of the therapeutic electric pulse and the charge balancing electrical pulse may be realized simply and cost-effectively by charging and discharging of capacitors.

In one embodiment the shape of the therapeutic electric pulse of one electrode is similar to the shape of the charge balancing pulses of the (N-1) other electrodes provided at the phase of the cycle, wherein similarity is meant in the mathematical sense comparing both shapes. However, the amplitude values of the therapeutic electrical pulse and the charge balancing electric pulses and their absolute values are different. In particular, the slope or form of the leading edge, the slope or form of the trailing edge and the slope or form of the plateau are similar, wherein the amplitude values of the charge balancing electric pulses and their absolute values are smaller than the amplitude value(s) of the therapeutic electric pulses.

The inventors have found that a rampart or non-rectangular pulse shape as described above permits influence over the recruitment order of axonal diameter, or allows more efficient charge utilization, or a combination of both caused by its shape different from the rectangular pulse shape. The invention provides a unique combination of multiphase therapy modes together with the above explained more advanced, non-rectangular pulse shape, and thereby creates an unexpected enhancement of the reverse recruitment effect as a result of asymmetric pulse repetition and neural network neighbor influence. The above device's therapy reduces perception in SCS while mitigating pain. This multiphase neurostimulation minimizes the recruitment of large diameter nerve fibers and provides distributed neuron recruiting compounding effect thus further reducing paresthesia. It improves therapeutic efficacy and/or reduces power consumption.

In one embodiment the device provides modified multiphase therapy where the therapeutic electric pulses and the charge balancing electric pulses consist of, for example:
a fast leading edge, a constant plateau, and an exponential or linear trailing edge that has the advantage to arrest the propagation of action potentials of large-diameter fibers, or
linear or exponential leading edge, a constant plateau, and an exponential or linear trailing edge; or
a fast or rapidly changing leading edge, (exponential) capacitive discharge stimulation shapes which preferably contain one or more sequential capacitive charges and discharges at the plateau between the first amplitude value and the second amplitude value and a trailing edge forming in one section an (exponential) capacitive discharge stimulation shape, as well, and in the other section a rectangular or linear curve.

In one embodiment, the device is configured to deliver the therapeutic electric pulse of one electrode such that it has a (preferably changing) plateau amplitude value I1, I2, ... IN specific to this electrode.

In one embodiment the device is configured to deliver the therapeutic electric pulses and charge balancing electric pulses such that the (different) therapeutic electric phases and/or the charge balancing phases are separated by inter-phase intervals. The frequency of the cycles may be below or equal to 1,500 Hz, in a further embodiment between 1,000 Hz and 1,500 Hz. In one embodiment the plateau amplitude of the therapeutic electric pulses (or therapeutic phases) is in the range from 0.5 mA to 20.0 mA, in a further embodiment in the range from 0.5 mA to 5.0 mA, and in a further embodiment in the range from 1.0 mA to 5.0 mA.

In one embodiment the device may be configured such that the plateau amplitude values of the charge balancing electric pulses is the (N-1)th part of the specific plateau amplitude value of the specific therapeutic electric pulse delivered in the same phase during one of the N therapeutic electrical pulses. In this embodiment the charge balancing electric pulses are preferably equally shared between the electrodes of the group of electrodes. With regard to the pulse shapes that are oscillating during the plateau section, the above applies to the average value over the whole plateau section of the pulse. Other distribution of charge balancing electric pulses across these (N-1) electrodes is possible, as well. Similarly, in one embodiment the device is configured such that during one phase each electrode of the group delivers an electrical pulse with a plateau amplitude value that establishes charge neutrality on each respective electrode based on pulses of the N-1 other phases of the same electrode. In a further embodiment, the device is configured such that during one phase each electrode of the N group delivers an electrical pulse with an amplitude value that establishes charge neutrality on each respective electrode based on residual charge from the other N phases.

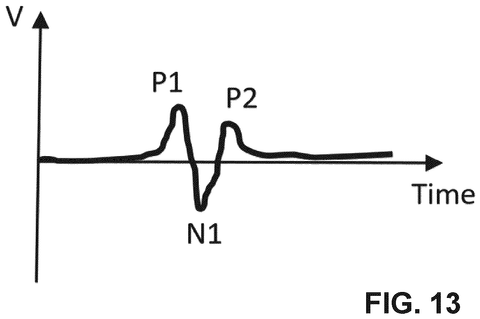

In one embodiment, in particular for use outside of a clinic, the device may be configured such that the plateau amplitude value of the specific therapeutic electric pulse of each electrode of the group is automatically adjusted using measurement of recorded evoked compound action potential (ECAP) waveform. The measurement results are used to determine activation thresholds and thereby the specific therapeutic electric pulse plateau amplitude for the respective electrode. Further, in this case the device may be configured such that ECAP waveform measurement is provided using at least one auxiliary electrode of the plurality of Z electrodes different from the group of N electrodes. Furthermore, the device may be configured such that the ECAP waveform measurement is provided in predefined time intervals and/or if the patient's body position and/or activity change is detected, for example by using an accelerometer contained within the device. Beyond manual programming, a preferred method of neurostimulation is thereby provided which enables periodic automatic adjustments of amplitude employs closed-loop stimulation (or periodic automatic adjustments of plateau amplitude via closed-loop stimulation). ECAPs are recorded, for example from dorsal column fibers, while therapy is delivered (leveraging on the embodiments described in US Patent 10,842,996 B2) and individual therapeutic electric phase amplitudes (or individual therapeutic electric pulse plateau amplitudes) may be adjusted to maintain the sensed ECAPs amplitudes within a therapeutic sub-perception window. This sub-perception window is typically only up to approximately 10 µV in amplitude, when stimulating with tens of Hz frequencies, whereas the perception window typically extends to less than 35 µV (but may also be 35 µV or more). Any generated ECAP amplitude above 35 µV may be associated with undesirable stimulation for the patient. This may be patient dependent though and may require calibration to adjust the (plateau) amplitudes. The ECAP amplitude is defined as the voltage difference between the second positive peak P2 and the negative peak P1 as shown in Fig. 13.

In one embodiment the device may be configured such that for assessing the plateau amplitude of the specific therapeutic electric pulse of each electrode of the group, the specific activation threshold for each electrode is determined, wherein the (plateau) amplitude of one specific electrode of the group is a pre-defined part of the measured specific activation threshold of this electrode. The 'perception threshold' or 'activation threshold' refers to the stimulation amplitude which is just strong enough to induce action potentials (e.g. neural response threshold, ECAP amplitude in the perception window as described before). Previous reports in prior art demonstrate that action potential thresholds typically coincide with perception threshold. Furthermore, action potentials generation is a pre-requisite for recording ECAPs. The device may be configured such that the determination of the specific activation threshold of each electrode of the group may comprise in a first step a rough titration by ramping the therapeutic electric phase amplitudes (or therapeutic electric pulse plateau amplitudes) up on all N electrodes of the group simultaneously until the activation threshold is reached at one electrode. Then, the therapeutic electric pulse plateau amplitude on all N electrodes is reduced until below this activation threshold. Afterwards, for each electrode of the group, the plateau amplitude is increased separately in order to detect the specific activation threshold of each electrode of the group. For sub-perception therapy the stimulation (plateau) amplitude of the therapeutic electric pulse is programmed to a pre-defined part of 40% to 60%, for example 50%, of the determined activation threshold on each electrode. The above procedure may be repeated for different activity changes or positions of the patient's body.

In a further embodiment, during any of the N phases, the device casing, in particular the pulse generator casing, may source or sink current to provide balance to the net currents of the active electrodes.

The object is further solved by a method for neurostimulation of a patient's body using a plurality of Z electrodes, wherein for the number of the plurality of electrodes Z ≥ 3 applies (or wherein the number of the plurality of electrodes Z is equal to or higher than 3), wherein during one cycle each electrode of a group of N electrodes (N ≤ Z and if Z = 3 then N = Z) delivers a set of electric pulses including one therapeutic electric pulse having an amplitude I1, I2, ... IN and a number of (N-1) charge balancing electric pulses, wherein the charge balancing electric pulses each have a polarity being opposite a polarity of the therapeutic electric pulse, and wherein each therapeutic electric pulse and each charge balancing electric pulse has a rampart or non-rectangular shape.

This shape may have a leading edge, a trailing edge and a plateau between the leading edge and the trailing edge, wherein at least one section of the leading edge and/or the trailing edge forms a linear curve or an exponential curve, and wherein the plateau has a changing or constant amplitude.

In one embodiment of the method, the plateau has the changing amplitude, and the changing amplitude comprises at least two periodic oscillations between a first amplitude value and a second amplitude value, the absolute values of the first and the second amplitude values are greater than zero and the first amplitude value and the second amplitude value have the same sign.

In one embodiment of the method, a current of the therapeutic electric pulse is equal to a sum of currents of the charge balancing electric pulses.

In one embodiment of the method, the rampart or non-rectangular shape of the therapeutic electric pulses and the charge balancing electric pulse comprises
a) the leading edge having a fast (rising) edge shape, an exponential shape or a linear shape (linear slope), and
b) the trailing edge having a linear shape (linear slope) or a section running exponentially.

In one embodiment of the method, each therapeutic electric pulse and each charge balancing electric pulse has the rampart shape, wherein the rampart shape of the charge balancing electric pulse comprises
a) the leading edge having a rectangular shape, an exponential shape or a linear shape, and
b) the trailing edge having a linear shape or a section running exponentially.

In one embodiment of the method, the therapeutic electric pulse of one electrode has a plateau amplitude value I1, I2, ... IN specific to this electrode. In one embodiment, for example, the plateau amplitude value of the specific therapeutic electric pulse of each electrode of the group (102.a, 102.b, 102.c) is automatically adjusted using ECAP waveform measurements.

In one embodiment of the method, during one phase each electrode of the electrodes of the group delivers an electrical pulse with an plateau amplitude value that establishes charge neutrality on each respective electrode based on pulses of the N-1 other phases of the same electrode.

In one embodiment of the method, during one phase each electrode of the electrodes of the group delivers an electrical pulse with an amplitude value that establishes charge neutrality on each respective electrode based on residual charge from the other N phases of the same electrode.

The advantages of the inventive method and above embodiments of the method are already explained above in connection with the inventive system (or device) and its embodiments. It is therefore referred to the above explanation.

The herein and above disclosed neurostimulation system (and device) or method may particularly be used for spinal cord stimulation (SCS), but deep brain stimulation (DBS), pelvic stimulation, gastric stimulation, or peripheral nerve stimulation (PNS) may be provided with the above (disclosed) system (and device) or method, as well.

The above and herein disclosed system (and device) and method provide a unique combination of multiphase therapy modes and certain non-rectangular pulse shapes, together, which creates an unexpected enhancement of the reverse recruitment effect as a result of asymmetric pulse repetition and neural network neighbour influence.

Furthermore, detailed embodiments and features of the present invention will be described below with reference to schematic drawings, wherein
- Fig. 1: shows an embodiment of a neurostimulation system within a patient's body being part of a SCS implantable system,
- Fig. 2: shows a wiring diagram of an implantable pulse generator (IPG) front-end for the SCS device of Fig. 1,
- Fig. 3: shows an example of positioned electrodes for SCS-therapy delivery using the device of Fig. 1,
- Fig. 4: depicts a flow diagram of a titration method for specific (plateau) amplitudes for therapeutic electric pulses for different electrodes,
- Fig. 5: shows a first embodiment of one cycle of period T of the neurostimulation method with 3 electrodes (current vs. time),
- Fig. 6: shows the rampart or non-rectangular pulse shape of the embodiment of Fig. 5 in more detail (current vs. time),
- Fig. 7: depicts another rampart or non-rectangular pulse shape in more detail (current vs. time),
- Fig. 8: shows a wiring diagram of an SCS device providing the pulse shapes shown in Fig. 6 and 7 to one electrode as a therapeutic electric pulse and a charge balancing pulse,
- Fig. 9: shows a second embodiment of one cycle of period T of the neurostimulation method with 3 electrodes (current vs. time),
- Fig. 10: shows a wiring diagram of an SCS device providing the pulse shapes shown in Fig. 11 and 12 to electrodes as a therapeutic electric pulse and a charge balancing pulse,
- Fig. 11: shows a third embodiment of one cycle of period T of the neurostimulation method with 3 electrodes (current vs. time),
- Fig. 12: depicts a fourth embodiment of one cycle of period T of the neurostimulation method with 3 electrodes (current vs. time), and
- Fig. 13: a voltage profile defining the ECAP amplitude.

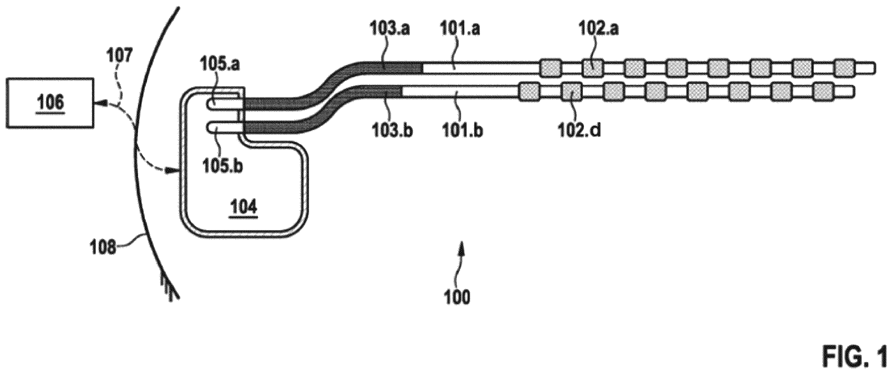

Fig. 1 illustrates an example of an implantable system 100 for spinal cord stimulation (SCS) as one example of a neurostimulation device. Such system includes first and second implantable percutaneous leads 101.a and 101.b that are implanted into a targeted location in the epidural space. Such lead may be replaced by a paddle lead (surgical lead) or other type of SCS lead.

The distal portion of the leads 101.a and 101.b incorporate a plurality of electrodes 102.a and 102.d respectively. Octal percutaneous leads 101.a and 101.b (i.e. each lead has eight electrodes 102) are shown in the example illustrated in Fig. 1. Each electrode 102.a, 102.d is connected to an insulated wire (not shown) that run inside flexible insulated carriers 103.a and 103.b. These carriers 103 get tunnelled during implantation to the vicinity of the implantable pulse generator (IPG) 104 that is typically implanted subcutaneously in the patient's lower abdominal or gluteal region. Carriers 103.a and 103.b terminate proximally in connectors 105.a and 105.b respectively that are then inserted into the header of the IPG 104 to allow conducting electrical charge to electrodes 102.a, 102.d.

The IPG 104 may communicate with one external device 106 through suitable radio frequency (RF, e.g. MICS-band or low-energy Bluetooth^{®}) or an inductive link 107 through the patient's skin 108. The external device 106 may include a computer such as clinician programmer or a patient remote, or an external charger among others. An external charger may send power transcutaneously though an inductive link 107 for battery recharge if the IPG 104 is powered by a secondary battery.

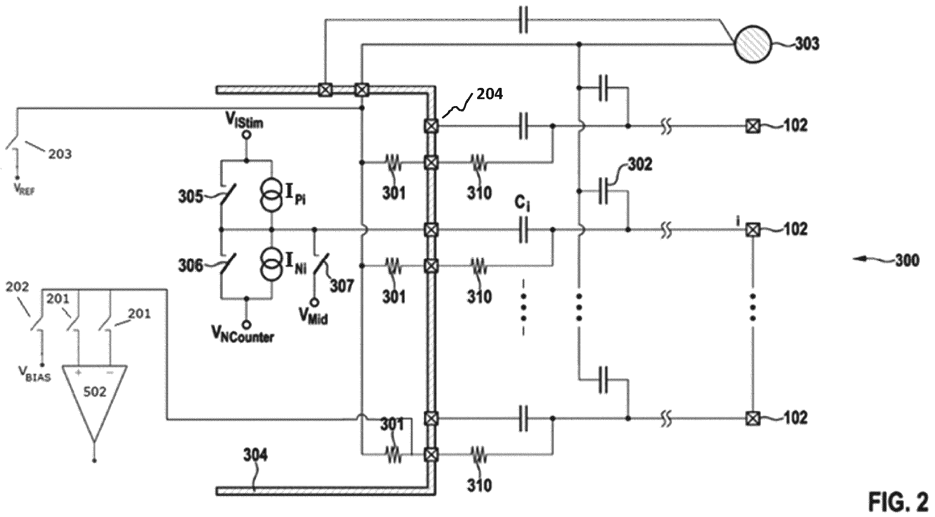

The electrodes 102.a, 102.d (in the following electrodes 102) are electrically driven by a front-end 300 which is located in the IPG 104. The front-end 300 is shown in Fig. 2. Component Cᵢ represents the DC blocking capacitor in series with each of the electrodes 102 traditionally employed to deliver electrical stimulation.

Resistors 301 in Fig. 2 are bleeding resistors (hundreds of kΩ), placed in star configuration, typically utilized in IPG's front-end 300 for passive charge neutrality. Capacitors 302, also in star configuration, provide filtering against electromagnetic interference (transitory voltage suppression protections, such as against external defibrillation pulses and electrostatic discharges, are not shown). The common node of both resistors 301 and capacitors 302 star configurations are connected to the conductive area 303 of the case of IPG 104.

An application specific integrated circuit (ASIC) 304 provides five controllable elements for biphasic stimulation where only one may be active at any time when the respective electrode 102 is utilized for therapy delivery. Current I_{Pi} permits sourcing current through an electrode 102 from the programmable voltage V_{IStim} whereas current I_{Ni} permits sinking current to a programmable voltage V_{NCounter}, which may be system ground Vss, as desired. Having sourcing and sinking currents independently controllable at each electrode 102 (shown only for one electrode 102) permits delivering simultaneous multi-electrode SCS therapy with active charge balancing. Analog switches 305, 306 permit connecting an electrode 102 to either V_{IStim} or V_{NCounter}, respectively, when currents of only one type are to be applied. Analog switches 307, referenced to a mid-voltage V_{Mid}, permit passive charge balancing. Voltage V_{Mid} may be any voltage between V_{IStim} and Vss including them. Resistors 310 may be added to limit the current in the presence of externally-generated fields (e.g. defibrillation). Conductive area 303 of IPG 104 case may also be connected to voltage V_{REF}, via analog switch 203, to deliver multiphase therapy (Described in more details within US application with application number US 63/168,604).

Analog switches 201 permit connecting any electrode 102 (shown only for one electrode 102) to an ECAP recording front-end 502. An electrode 102 can also be connected to a low-noise DC voltage V_{BIAS} during ECAP recording via analog switch 202 to fix a common mode voltage for recording. Embodiments for ECAP recording are further disclosed in US Patent 10,183,168 B2.

The IPG 104 of the embodiment is capable of delivering multi-modality SCS therapy as explained in the following.

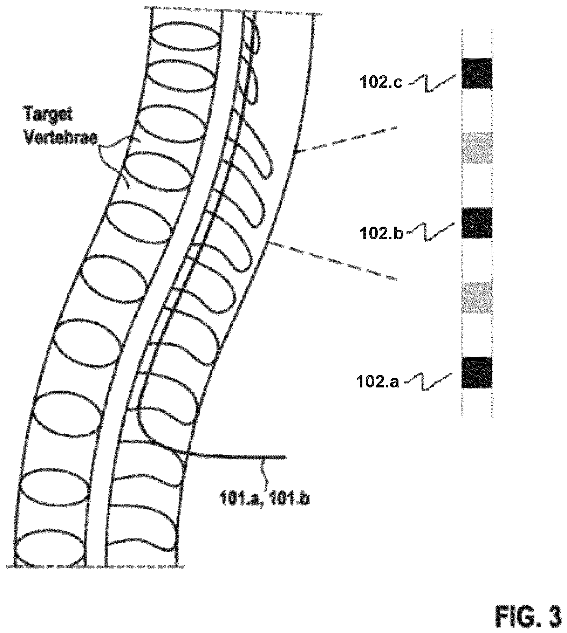

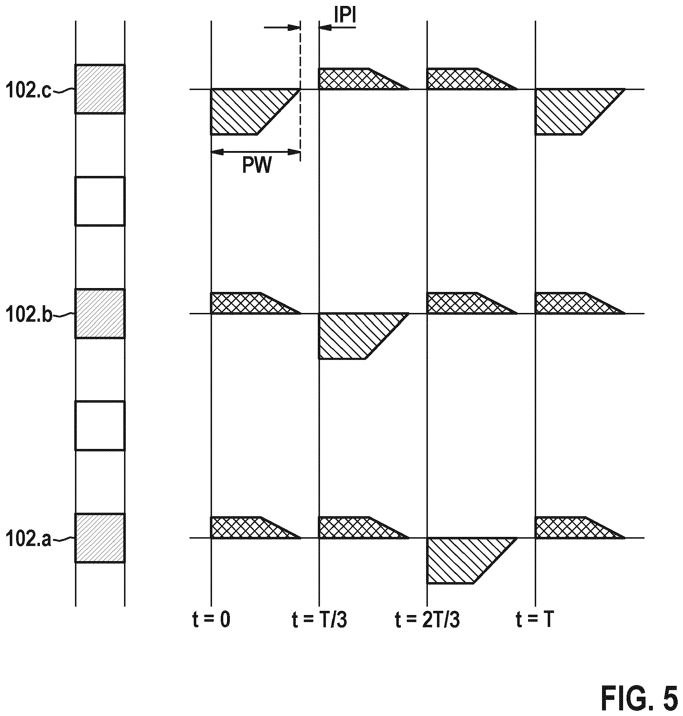

Without losing generality, an embodiment of a therapy approach based on the novel multiphase stimulation waveform of the present invention, as presented in Fig. 5, is described for the treatment of chronic back and leg pain, e.g. via spinal cord stimulation (SCS). Such therapy utilizes three (N = 3) electrodes 102, namely 102.a, 102.b, and 102.c as shown in Fig. 3 and 5. Leads 101.a and 101.b are implanted and positioned so that electrodes 102.a, 102.b, and 102.c of one lead 101.a in the thoracic region may be utilized for therapy.

In the example the first therapeutic electric pulse (TEP) of the novel multiphase stimulation waveform is that of electrode 102.c (see obliquely striped pulse in Fig. 5) which delivers the first specific cathodic TEP pulse with rampart or non-rectangular shape by the first electrode 102.c. The two other electrodes 102.b and 102.a deliver the anodic charge balancing electric pulses each with similar rampart or non-rectangular shape (see pulses of the same phase with the checkerboard pattern in Fig. 5). In the next phase electrode 102.b delivers the second previously determined cathodic TEP with rampart or non-rectangular shape and the other electrodes 102.c, 102.a the charge balancing electric pulses each having similar rampart or non-rectangular shape. After this, in the third phase the electrode 102.a delivers the third previously determined cathodic TEP having rampart or non-rectangular shape and the other electrodes 102.c, 102.b the charge balancing electric pulses having each similar rampart or non-rectangular shape. The first phase with the first TEP and the second phase with the second TEP and the third phase with the third TEP form one cycle and are repeated.

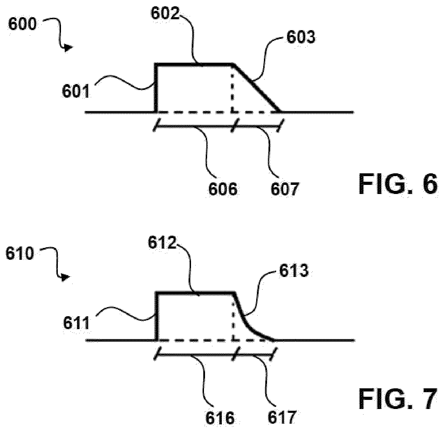

Each of the TEPs and the charge balancing electric pulses of the cycle depicted in Fig. 5 have a rampart or non-rectangular shape 600 as shown in Fig. 6 but with different (plateau) amplitudes. The pulse has a fast leading edge 601 (µs), followed by a constant plateau 602 of specific constant amplitude established over a time period 606 and a linear trailing edge (slope) 603. The specific amplitude of plateau 602 is ramped down along trailing edge 603 linearly reaching zero after a time period 607. The added time periods 606 and 607 form the Pulse Width (PW) shown in Fig. 5. The time period 606 of the plateau 602 may typically be tens of µs to a ms. The same length has time period 607.

One embodiment of timing parameters (see Fig. 5) is 30 µs and 140 µs for PW and Inter-pulse Intervals (IPI), respectively. This results in an equivalent frequency f, for the therapeutic phase at each electrode 102.a to 102.c, slightly above 1,450 Hz. The PW example range is from 15 µs to 1,000 µs whereas that of IPI may start from tens of µs to hundreds of µs or even a few thousand µs.

Another embodiment of a TEP and charge balancing pulse form 610 is depicted in Fig. 7. The pulse has a fast leading edge 611, a constant plateau (amplitude) 612 and an exponential decay at its trailing edge 613. The time period 616 of the plateau 612 is similar to the time period 606, and the time period 617 of the exponentially decaying trailing edge 613 similar to the time period 607 of the pulse shape shown in Fig. 6.

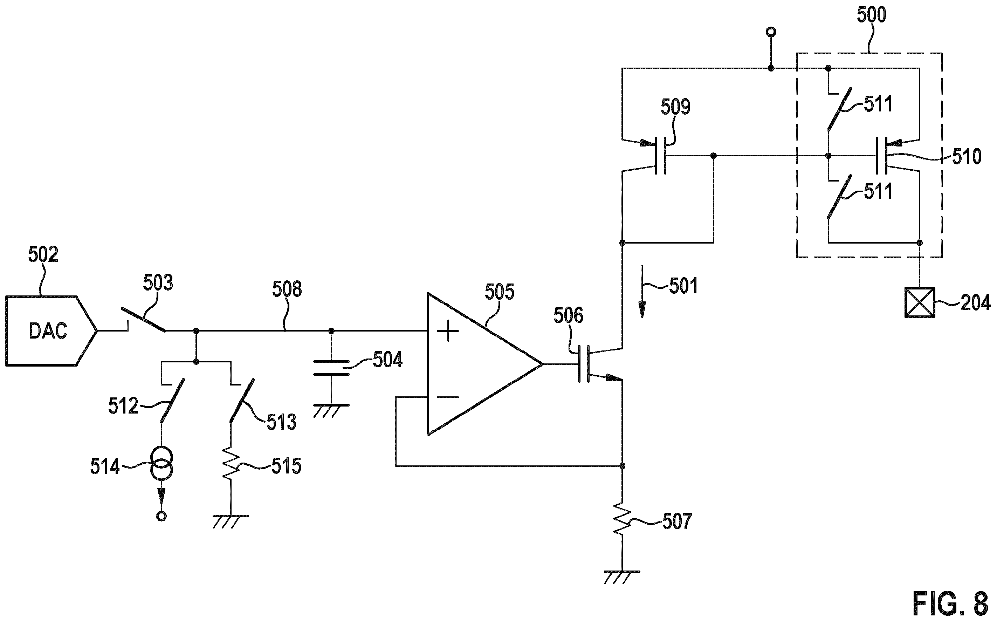

Typically, stimulation current sources for the electric pulse forms shown in Fig. 6 and 7 are implemented via a current digital-to-analog (DAC) converter 500 that drive the electrodes 102 via outputs 204 of the ASIC 304. This is illustrated in Fig. 8. A current reference 501 is generated using a voltage DAC 502, an analog switch 503, a capacitor 504, an operation amplifier 505, a transistor 506, and resistor 507. The DAC 502 imposes a voltage 508 in the non-inverting input of operational amplifier 505. The closed-loop connection of such operational amplifier 505 via transistor 506 forces the non-inverting input of 505 to follow voltage 508 resulting in reference current 501 being equal to voltage 508 divided by the value of resistor 507. Such reference current is mirrored via transistor 509 to the output DAC 500. In this example the DAC 500 is a 12-bit DAC implemented via transistors 510 and analog switches 511. Each electrode 102 is associated with a DAC 500. The output of DAC 500 connects to output 204 of the ASIC 304 that drives each electrode 102 via the DC blocking capacitors Cᵢ.

To generate the linear or exponential falling edges 603, 613 of durations 607 and 617 respectively, analog switch 503 is opened and either analog switch 512 or 513 closed respectively. Analog switch 512 permits discharging capacitor 504 via current sink 514 to generate a linear falling (slope) trailing edge 603 extending over the time period 607. Analog switch 513, on the other hand, permits discharging capacitor 504 via resistor 515 to implement the exponential decay at trailing edge 613 extending over time period 617. Although not shown, a bank of resistors 515 or capacitors 504 can be utilized to change time period 617. Different values of current 514 permit adjusting the linear falling edge time period 607.

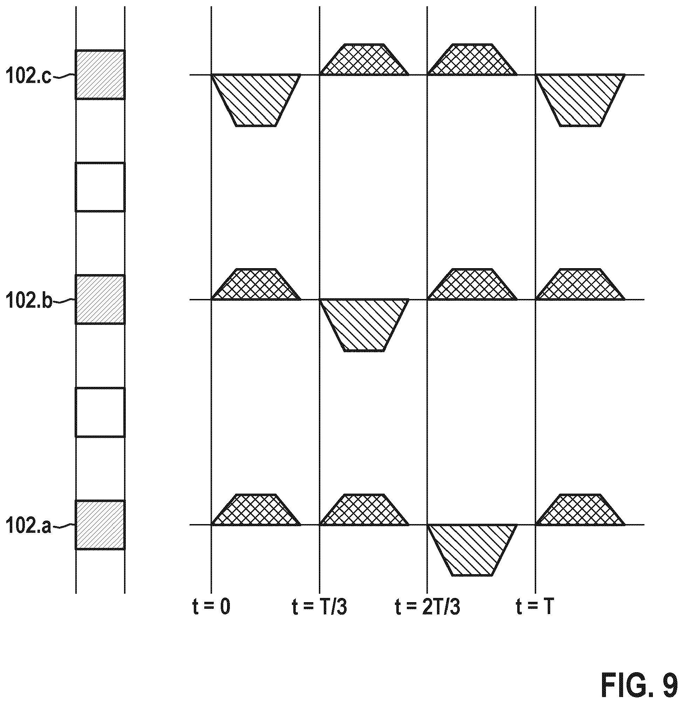

Fig. 9 shows another multiphase waveform embodiment wherein both the therapeutic electric pulse TEP (striped pulse) and the charge balancing electric pulse (checkerwork pulse) have a rampart or non-rectangular shape. Each pulse has a linear increasing or sloped leading edge which further reduces the preferential activation of large diameter fibers. These pulses are generated in the same manner and with the same electronics as the pulses shown in Fig. 5 and 6. With regard to Fig. 5, analog switch 516 and current source 517 can be used to generate the linear leading edge. As in previous embodiments, the modified TEP is generated by imposing return currents in the other electrodes 102, for example, when electrode 102.c delivers its TEP electrodes 102.b, 102.a are actively driven with current sources of the charge balancing electric pulses with rampart or non-rectangular shape.

In another embodiment, each of the pulses shown in Fig. 9 may be provided with opposite polarity but with similar rampart or non-rectangular pulse shape. This waveform presents distributed therapeutic compounding effects provided by the TEPs as they follow physiologic neurons burst firing, depending on the electrodes 102.a to 102.c distances.

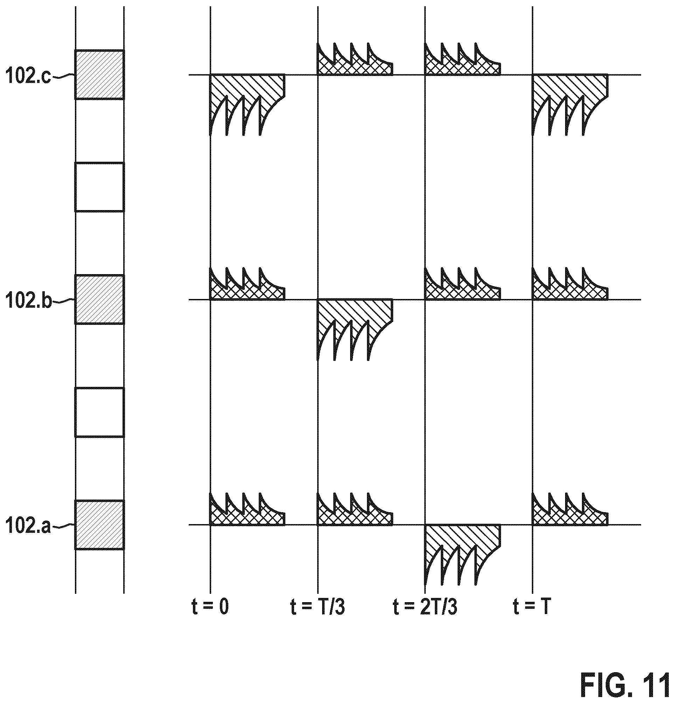

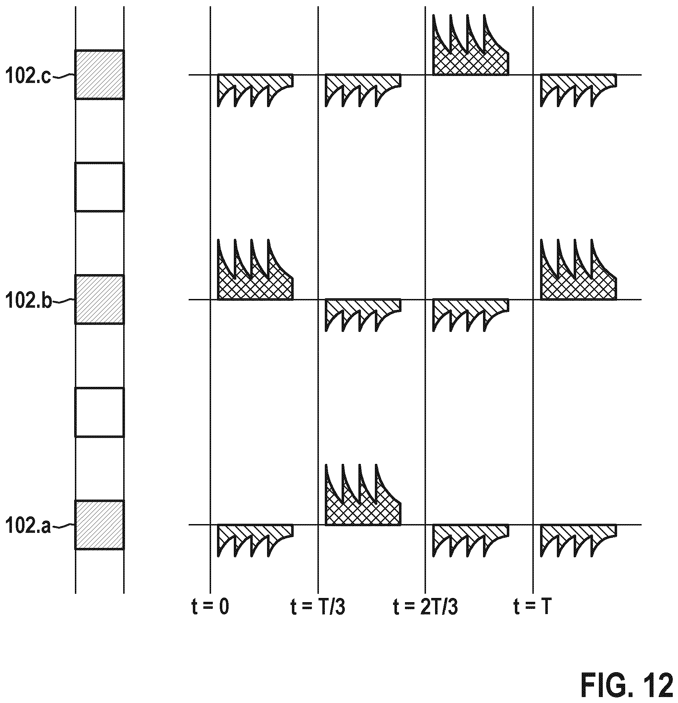

Fig. 11 shows another multiphase waveform embodiment utilizing multiple TEPs with another rampart or non-rectangular shape. The charge balancing electric pulses have the same rampart or non-rectangular shape, wherein the amplitudes are different as shown. Each pulse comprises a fast leading edge, a plateau having a periodic oscillation with an exponential decay from a first, higher amplitude to a second, lower amplitude and a subsequent fast increase to the first amplitude. The trailing edge consist of an exponential decay section from the first amplitude to zero. This multiple capacitive discharge pulse scheme enables efficient charge coupling from the IPG control circuit to a target neural population. In order to realize this pulse shape capacitors are charged to a target voltage, and sequentially discharged in a burst fashion through the target electrodes 102.a, 102.b, 102.c. The capacitors are coupled to the electrodes 102 via analog switches such that the connection is made through the switches when a TEP is desired. Similar to previous embodiments, capacitors to be discharged are simultaneously connected to return electrodes 102 for charge balancing electric pulses so the combined discharge currents added generates the TEP at the desired electrode 102. For example, if electrode 102.c delivers its TEP, electrodes 102.b, 102.a are connected to the capacitors to be discharged. Analog switch connections for capacitive charge and discharge are known to those familiar with the state of the art, and have been described previously in document US Patent 11,071,867 for a galvanic communication scheme. Although neither Fig. 11 nor Fig. 12 illustrates them, pauses (i.e. time periods in which no charge is delivered) may be present in between consecutive discharges at the plateau to elicit the required compounding effect. In a preferred embodiment such pause is 1 ms.

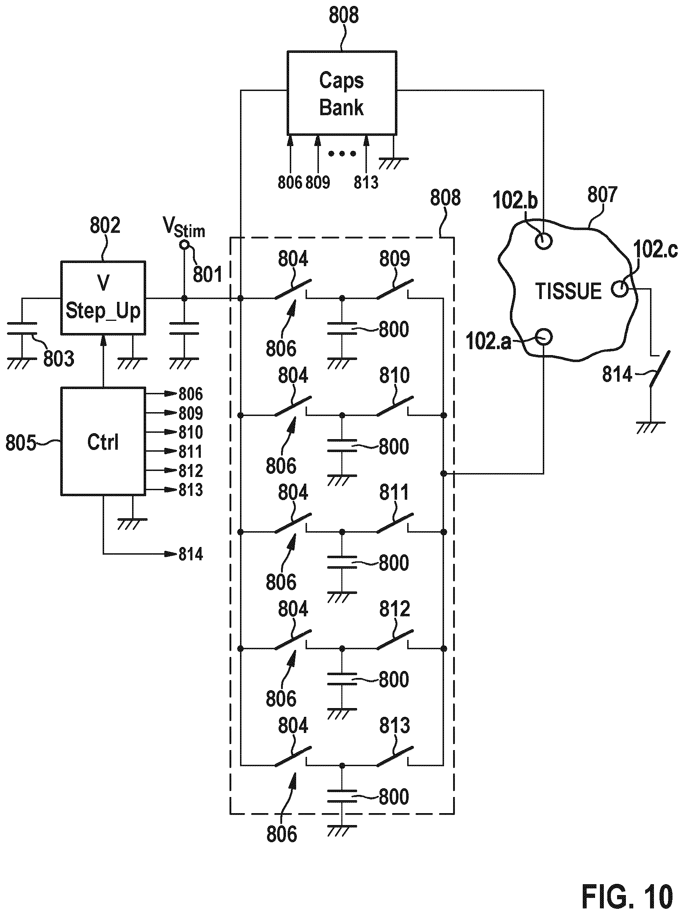

To deliver the first phase of the therapy shown in Fig. 11 (delivery of the other phases will be similar), in one embodiment, five (5) 150 nF nominal capacitors 800 are charged in parallel to a voltage VStim 801 (e.g. up to 16 V), for example generated via a voltage step-up (V step-up block) 802 from the battery 803 of the implantable pulse generator (IPG), as illustrated in Fig. 10. To charge the capacitors 800, analog switches 804 are closed by the control logic (Ctrl block) 805 of the IPG via common signal 806 when there is no need to deliver charge to tissue 807. This capacitor bank 808 (Caps Bank block) is associated with electrode 102.a to deliver a charge balancing electric pulse (pulse at t = 0) with the rampart or non-rectangular shape shown in Fig. 11. To deliver the charge balancing electric pulse, analog switches 809..813 are sequentially closed to deliver this charge. Electrode 102.b, on the other hand, has another Caps Bank 808 associated with it to simultaneously deliver charge with 102.a with the above described rampart or non-rectangular shape as another charge balancing pulse (see Fig. 11). For current to circulate through tissue 807, analog switch 814 is closed by Ctrl 805 connecting electrode 102.c to system ground thereby delivering TEP with the rampart or non-rectangular shape explained above and shown in Fig. 11.

In yet another embodiment, the waveform shown in Fig. 12 presents distributed therapeutic compounding effects provided by TEPs, as they follow physiologic neurons burst firing, depending on the electrodes 102.a to 102.c distances. The charge balancing electric pulses and the TEPs are similar to the pulses shown in Fig. 11 but with opposite polarity.

Figs. 5, 9, 11 and 12 disclose a novel combination of multiphase stimulation using pulses with a rampart or non-rectangular shape. This combination has the unique benefit of manipulating neural recruitment such that, for example, a cathodic phase with periodic oscillations, or one long cathodic phase which is longer than the anodic phase of a given stimulating electrode 102 providing an enhanced recruitment effect in two ways: 1) the oscillating or longer cathodic phase further enhances recruitment of smaller diameter fibers which are slower to react to external stimuli, and 2) at each phase of stimulation, there are at least two neural regions under active electrodes in the cathodic phase. For example, a time t = T/3 in Fig. 12, the neural tissue adjacent to electrodes 102.c and 102.b is being simultaneously stimulated. This simultaneous stimulation has the effect of enhancing synaptic connections between the stimulated regions as a result of synchronous activation, an effect known as Hebbian Plasticity Training.

One embodiment of charge balance, in at least one of the Inter-pulse Intervals IPI, is performed by closing switches 307 (see Fig. 2) for the participating electrodes 102.a to 102.c. This avoids voltage runaway in the DC blocking capacitors Cᵢ of the mentioned electrodes that may be caused by mismatches in the generation of the different I/2 (or I/(N-1, if N is different than 2) among the different electrode drivers. It also keeps the electrode 102.a, 102.b and 102.c potentials within acceptable ranges for continuous therapy delivery.

The TEP plateau or oscillating amplitudes may be programmable in the same range described before, i.e. from 0.5 mA to 20.0 mA, in a further embodiment in the range from 0.5 mA to 5.0 mA. In case the current is oscillating, the TEP amplitude value described in the previous sentence is the maximum amplitude. The maximum charge injected in any TEP may be also limited by the IPG 104 to 10 µC to avoid tissue and electrode damage.

As explained above the IPG 104 comprises N = 3 electrodes for TEP, each of the three electrodes undergoes a recurring pattern of an electrode specific TEP with a current plateau amplitude I and a series of (N-1) charge balancing electric pulses, which pass an inverted current plateau amplitude I of the specific TEP delivered at the same time, for example distributed with equal weight (I/(N-1)). Each therapeutic electric phase TEP (see e.g. obliquely striped pulse in Fig. 5) is timely aligned with one charge balancing electric pulse (see pulse with checkerboard pattern) of each of the other (N-1) electrodes such that in the cycle only one TEP occurs at one time. After every electrode 102.a..102.c (in the example) is cyclically-passed one TEP, the cycle starts again with the first of the N electrodes (see e.g. last column of Fig. 5 showing the amplitude levels for each electrode 102.a, 102.b and 102.c).

Hence, in the example depicted in Fig. 5 using three active electrodes with a Rotating Electrodes therapy design that uses rampart or non-rectangular TEPs with a linear falling slope (cathodic stimulation in the example), the therapy rotates across each individual electrode 102.a, 102.b, 102.c during each stimulation cycle while the remaining two electrodes serve as the anodic return. The TEP (cathodic) of each individual electrode 102 is balanced by its participation in two charge balancing pulses (anodic).

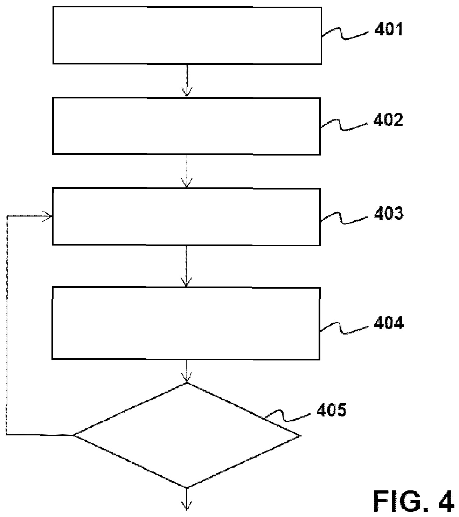

Fig. 4 shows one embodiment of a clinical flow diagram of the titration method to be used with independent therapeutic electric pulse (TEP) amplitudes (plateau or oscillating). In step 401 the TEP amplitudes for all N electrodes of the group delivering TEPs are ramped up with an equal amplitude until for one electrode 102 the activation threshold is reached. In step 402 the TEP amplitude for all N electrodes is reduced until below the activation threshold.

Following the above described rough titration close to threshold, the first TEP is presented to the patient at the first electrode and the amplitude is incremented to determine the activation threshold. The amplitude at the first electrode is increased until the specific activation threshold is reached (step 403). After the specific activation threshold is reached, for this specific electrode, the TEP amplitude is reduced until below the specific activation threshold (step 404). This is then repeated for each electrode 102 of the group of N electrodes for therapy. For that, step 405 asks whether there is another electrode (and repeats steps 403 and 404 if there is another electrode for TEP delivery). Finally, after all N electrodes are titrated, the so determined individual activation thresholds are used to set the TEP amplitude of each electrode 102 of the group independently.

## Claims

1. A system for neurostimulation (100) of a patient's body, wherein the system comprises a plurality of Z electrodes (102, 102.a, 102.b, 102.c, 102.d) and a device (104), wherein for the number of the plurality of electrodes Z ≥ 3 applies, wherein the device (104) is configured to deliver via each electrode of a group of N electrodes (N ≤ Z and if Z = 3 then N = Z) a set of electric pulses including one therapeutic electric pulse having an amplitude I1, I2, ... IN, and a number of (N-1) charge balancing electric pulses during one cycle, wherein the charge balancing electric pulses each have a polarity being opposite a polarity of the therapeutic electric pulse, and wherein each therapeutic electric pulse and each charge balancing electric pulse have a non-rectangular shape, wherein the non-rectangular shape has a leading edge, a trailing edge and a plateau between the leading edge and the trailing edge,
**characterized in that**
the plateau has a constant amplitude, and wherein
the leading edge has a fast edge shape, an exponential shape or a linear slope, and
the trailing edge has a linear slope or a section running exponentially.

2. The system (100) of claim 1, wherein the plateau has the changing amplitude, and the changing amplitude comprises at least two periodic oscillations between a first amplitude value and a second amplitude value, the absolute values of the first and the second amplitude values are greater than zero and the first amplitude value and the second amplitude value have the same sign.

3. The system (100) of any of the previous claims, wherein a current of the therapeutic electric pulse is equal to a sum of currents of the charge balancing electric pulses.

4. The system of any of the previous claims, wherein device is configured to deliver the therapeutic electric pulse of one electrode such that it has a plateau amplitude value I1, I2, ... IN specific to this electrode.

5. The system (100) of claim 4, wherein the device (104) is configured such that the plateau amplitude value of the specific therapeutic electric pulse of each electrode of the group (102.a, 102.b, 102.c) is automatically adjusted using evoked compound action potential (ECAP) waveform measurement.

6. The system (100) of any of the previous claims, wherein the device (104) is configured such that during one phase each electrode of the group delivers an electrical pulse with a plateau amplitude value that establishes charge neutrality on each respective electrode based on pulses of the N-1 other phases of the same electrode.

7. The system (100) of any of the previous claims, wherein the device (104) is configured that at the end of the cycle, a passive balance phase is incorporated, wherein the passive balance phase is configured to return the plurality of electrodes to a baseline low level of charge.

8. The system (100) of any of the previous claims, wherein the device (104) comprises a pulse generator (104), wherein the pulse generator comprises an application specific integrated circuit (ASIC).

9. The system (100) of any of the previous claims, wherein the plurality of electrodes is located at a distal portion of at least one implantable percutaneous lead.

10. The system (100) of any of the previous claims, wherein the plurality of electrodes is located at an implantable a paddle lead.

11. The system (100) of any of the previous claims, wherein the system (100) is configured to be part of a spinal cord stimulation (SCS) implantable system.

## Patentansprüche

1. System zur Neurostimulation (100) des Körpers eines Patienten, wobei das System eine Vielzahl von Z Elektroden (102, 102.a, 102.b, 102.c, 102.d) und eine Vorrichtung (104) umfasst, wobei für die Anzahl der Vielzahl von Elektroden Z≥3 gilt, wobei die Vorrichtung (104) dazu konfiguriert ist, über jede Elektrode einer Gruppe von N Elektroden (N≤Z und wenn Z=3, dann N=Z) einen Satz von elektrischen Impulsen, einschließlich eines therapeutischen Impulses mit einer Amplitude I1, I2,... IN und einer Anzahl an (N-1) elektrischen Ladungsausgleichsimpulsen während eines Zyklus, zu liefern, wobei die elektrischen Ladungsausgleichsimpulse jeweils eine Polarität entgegengesetzt zu einer Polarität des therapeutischen elektrischen Impulses aufweisen und wobei jeder therapeutische elektrische Impuls und jeder elektrische Ladungsausgleichsimpuls eine nicht rechteckige Form aufweisen,
wobei die nicht rechteckige Form eine Vorderflanke, eine Hinterflanke und ein Plateau zwischen der Vorderflanke und der Hinterflanke aufweist,
**dadurch gekennzeichnet, dass**
das Plateau eine konstante Amplitude aufweist, und wobei
die Vorderflanke eine Form einer schnellen Flanke, eine exponentielle Form oder eine lineare Steigung aufweist und die Hinterflanke eine lineare Steigung oder ein Teilstück, das exponentiell verläuft, aufweist.

2. System (100) nach Anspruch 1, wobei das Plateau die sich verändernde Amplitude aufweist und die sich verändernde Amplitude mindestens zwei periodische Schwingungen zwischen einem ersten Amplitudenwert und einem zweiten Amplitudenwert umfasst, die absoluten Werte des ersten und des zweiten Amplitudenwerts größer als null sind und der erste Amplitudenwert und der zweite Amplitudenwert das gleiche Vorzeichen aufweisen.

3. System (100) nach einem der vorhergehenden Ansprüche, wobei ein Strom des therapeutischen elektrischen Impulses gleich einer Summe aus Strömen der elektrischen Ladungsausgleichsimpulse ist.

4. System nach einem der vorhergehenden Ansprüche, wobei die Vorrichtung dazu konfiguriert ist, den therapeutischen elektrischen Impuls einer Elektrode so zu liefern, dass er einen Plateauamplitudenwert I1, I2,... IN aufweist, der für diese Elektrode spezifisch ist.

5. System (100) nach Anspruch 4, wobei die Vorrichtung (104) derart konfiguriert ist, dass der Plateauamplitudenwert des spezifischen therapeutischen elektrischen Impulses jeder Elektrode der Gruppe (102.a, 102.b, 102.c) automatisch unter Verwendung einer Wellenformmessung durch evozierte Summenaktionspotentiale (ECAP) eingestellt wird.

6. System (100) nach einem der vorhergehenden Ansprüche, wobei die Vorrichtung (104) derart konfiguriert ist, dass während einer Phase jede Elektrode der Gruppe einen elektrischen Impuls mit einem Plateauamplitudenwert liefert, der zu einer Ladungsneutralität an jeder jeweiligen Elektrode basierend auf Impulsen der N-1 anderen Phasen der gleichen Elektrode führt.

7. System (100) nach einem der vorhergehenden Ansprüche, wobei die Vorrichtung (104) dazu konfiguriert ist, dass am Ende des Zyklus eine passive Ausgleichsphase integriert wird, wobei die passive Ausgleichsphase dazu konfiguriert ist, die Vielzahl von Elektroden zu einem niedrigen Ausgangsladungspegel zurückzuführen.

8. System (100) nach einem der vorhergehenden Ansprüche, wobei die Vorrichtung (104) einen Impulsgenerator (104) umfasst, wobei der Impulsgenerator eine anwendungsspezifische integrierte Schaltung (ASIC) umfasst.

9. System (100) nach einem der vorhergehenden Ansprüche, wobei sich die Vielzahl von Elektroden an einem distalen Abschnitt von mindestens einer implantierbaren perkutanen Leitung befindet.

10. System (100) nach einem der vorhergehenden Ansprüche, wobei sich die Vielzahl von Elektroden an einer implantierbaren Paddle-Leitung befindet.

11. System (100) nach einem der vorhergehenden Ansprüche, wobei das System (100) dazu konfiguriert ist, Teil eines implantierbaren Systems zur Rückenmarkstimulation (SCS) zu sein.

## Revendications

1. Système de neurostimulation (100) du corps d'un patient, dans lequel le système comprend une pluralité de Z électrodes (102, 102.a, 102.b, 102.c, 102.d) et un dispositif (104), dans lequel pour le nombre de la pluralité d'électrodes Z ≥ 3 s'applique, dans lequel le dispositif (104) est configuré pour distribuer par l'intermédiaire de chaque électrode d'un groupe de N électrodes (N ≤ Z et si Z = 3 alors N = Z) un ensemble d'impulsions électriques incluant une impulsion électrique thérapeutique ayant une amplitude I1, I2, ... IN, et un nombre de (N-1) impulsions électriques d'équilibrage de charge pendant un cycle, dans lequel les impulsions électriques d'équilibrage de charge ont chacune une polarité qui est opposée à une polarité de l'impulsion électrique thérapeutique, et dans lequel chaque impulsion électrique thérapeutique et chaque impulsion électrique d'équilibrage de charge ont une forme non rectangulaire, dans lequel la forme non rectangulaire a un flanc de pointe, un flanc de fuite et un plateau entre le flanc de pointe et le flanc de fuite, **caractérisé en ce que**
le plateau a une amplitude constante, et dans lequel
le flanc de pointe a une forme de flanc serré, une forme exponentielle ou une pente linéaire, et le flanc de fuite a une pente linéaire ou une section évoluant exponentiellement.

2. Système (100) selon la revendication 1, dans lequel le plateau a une amplitude changeante, et l'amplitude changeante comprend au moins deux oscillations périodiques entre une première valeur d'amplitude et une seconde valeur d'amplitude, les valeurs absolues des première et seconde valeurs d'amplitude sont supérieures à zéro et la première valeur d'amplitude et la seconde valeur d'amplitude ont le même signe.

3. Système (100) selon l'une quelconque des revendications précédentes, dans lequel un courant de l'impulsion électrique thérapeutique est égal à une somme des courants des impulsions électriques d'équilibrage de charge.

4. Système selon l'une quelconque des revendications précédentes, dans lequel le dispositif est configuré pour distribuer l'impulsion électrique thérapeutique d'une électrode de telle sorte qu'elle a une valeur d'amplitude de plateau I1, I2, ... IN spécifique à cette électrode.

5. Système (100) selon la revendication 4, dans lequel le dispositif (104) est configuré de telle sorte que la valeur d'amplitude de plateau de l'impulsion électrique thérapeutique spécifique de chaque électrode du groupe (102.a, 102.b, 102.c) est automatiquement ajustée en utilisant une mesure de forme d'onde de potentiel d'action évoqué composé (ECAP).

6. Système (100) selon l'une quelconque des revendications précédentes, dans lequel le dispositif (104) est configuré de telle sorte que pendant une phase chaque électrode du groupe distribue une impulsion électrique avec une valeur d'amplitude de plateau qui établit une neutralité de charge sur chaque électrode respective en se basant sur les impulsions des N-1 autres phases de la même électrode.

7. Système (100) selon l'une quelconque des revendications précédentes, dans lequel le dispositif (104) est configuré de telle sorte qu'à la fin du cycle, une phase d'équilibre passif est incorporée, dans lequel la phase d'équilibre passif est configurée pour renvoyer la pluralité d'électrodes à un niveau de charge faible de ligne de base.

8. Système (100) selon l'une quelconque des revendications précédentes, dans lequel le dispositif (104) comprend un générateur d'impulsions (104), dans lequel le générateur d'impulsions comprend un circuit intégré spécifique à une application (ASIC).

9. Système (100) selon l'une quelconque des revendications précédentes, dans lequel la pluralité d'électrodes est située au niveau d'une partie distale d'au moins un câble percutané implantable.

10. Système (100) selon l'une quelconque des revendications précédentes, dans lequel la pluralité d'électrodes est située au niveau d'un câble à spatule implantable.

11. Système (100) selon l'une quelconque des revendications précédentes, dans lequel le système (100) est configuré pour faire partie d'un système implantable de stimulation de la moelle épinière (SCS).
